# EUROPEAN PATENT SPECIFICATION

(11) **EP 1 727 507 B1**
(45) Date of publication and mention of the grant of the patent: **28.07.2010**
(21) Application number: 05725052.4
(22) Date of filing: 08.03.2005
(51) Int. Cl.: A61K 6/00, A61K 6/06, A61K 6/083, B29C 67/24, C04B 26/02

(54) **POLYMER-BIOACTIVE CERAMIC/CEMENT HYBRID COMPOSITE**
HYBRID-KOMPOSIT AUS POLYMER/BIOAKTIVEM KERAMIK/ZEMENT
CÉRAMIQUE BIOACTIVE POLYMÉRIQUE/ CIMENT HYBRIDE COMPOSITE

(30) Priority: 09.03.2004 US 551738 P
(43) Date of publication of application: 06.12.2006
(73) Proprietor: DENTSPLY International Inc., York, PA 17405-0872 (US)
(72) Inventor: JEFFERIES, Steven, R., York, PA 17402 (US); PRIMUS, Carolyn, M., Bradenton, FL 34203 (US)
(74) Representative: Hartz, Nikolai
(86) International application number: PCT/US2005/007670
(87) International publication number: WO 2005/087178

(56) References cited:
- EP-A- 0 587 383
- WO-A-2005/039509
- US-A- 4 647 600
- US-A- 4 689 080
- OHAMA Y: "POLYMER-BASED ADMIXTURES" April 1998 (1998-04), CEMENT AND CONCRETE COMPOSITES, ELSEVIER APPLIED SCIENCE, BARKING,, GB, PAGE(S) 189-192 , XP000972515 ISSN: 0958-9465 the whole document
- VIZGIRDA, PAUL J; LIEWEHR FREDERICK R; PATTON, WILLIAM R; MCPHERSON, JAMES C; BUXTON T B: "A comparison of laterally condensed gutta-percha, thermoplasticized gutta-percha, and mineral trioxide aggregate as root canal filling materials" JOURNAL OF ENDODONTICS, vol. 30, no. 2, February 2004 (2004-02), pages 103-106, XP008048839 USA
- David L. Spencer: "Physical properties of a new mineral trioxide aggregate material" 2004, * abstract *

## Description

### TECHNICAL FIELD

The invention relates to a dental composite material. Specifically the invention relates to a polymer-bioactive ceramic, cement hybrid composite material.

### BACKGROUND OF THE INVENTION

Calcium-based, bioactive cements or particles have demonstrated the ability to facilitate hard and soft tissue healing in various dental applications. Materials such as BioGlass, Calcium Phosphate-Based Cements, Hydroxyapatite-based materials. Calcium Aluminate-Based cements, and portland cements, are just some of the calcium-based biomaterials that have been used in hard and soft-tissue repair. Specific dental applications of these cements include for example, pulp capping agents, pulpotomy agents, root repair material for breaches in the dentin and cementum, root end filling materials, and in selected instances, as bone grafting agents. These materials are water-based cements, which can react, in acidic, neutral, or basic aqueous environments to form cementitious structures.

US-A 4,647,600 discloses a dental cement composition for use as a pulp capping and living cement which does not contain a mineral trioxide aggregate.

While such materials have enjoyed widespread use and acceptance, drawbacks of these calcium-based, particulate cements is their slow reaction rate, a propensity for early moisture sensitivity and high solubility, and low early strength.

A need exists therefore, for a method and final conjugate structure which overcomes the low early strength, low surface hardness, slow setting, and high initial solubility of these calcium-based cements, while at the same time retaining the biocompatibility of these materials.

### SUMMARY OF THE INVENTION

The present invention provides novel polymer-infiltrated structures of calcium-based cements as defined by claim 1. The polymer-infiltrated structure stabilizes the surface of the set or setting calcium-based cement, preventing washout and stabilizing the restoration in its desired location.

### PREFERRED EMBODIMENTS FOR CARRYING OUT THE INVENTION

The present invention provides novel polymer-infiltrated structures of calcium-based cements as defined by claim 1. The polymer-infiltrated structure stabilizes the surface of the set or setting calcium-based cement, preventing washout and stabilizing the restoration in its desired location.

A variety of polymer-based materials may be used to infiltrate and reinforce the boundaries and surfaces of the calcium-based cement Water-soluble polymers of various types and compositions may be used. Alternatively, hydrophobic resin compositions, with or without the use of solvents (acetone, alcohol, etc.), carrier vehicles, or viscosity modifiers, may be deposited to, brushed on, sprayed on, or otherwise delivered to the surfaces or bulk of the calcium-based cement prior to setting or in its fully set state.

The surface infiltration of the cement permits stabilization of the cement before the cement is fully set, creating a unique calcium-containing, biomaterial-based hybrid complex that facilitates healing at one interface and while stabilizing the other surface or bulk properties for the cement with respect to sealing or physical properties. Thus, this materials complex becomes uniquely optimized with respect to physical stability and biocompatibility/bioactivity.

In another embodiment of the invention there is the development of a pre-reacted cement complex, reduction of the complex to particles or elements of a defined size and/or shape, followed by infiltration or complexation with an appropriate polymer material. One illustration of this approach involves the formation of a set, calcium-based, water-based cement, followed by milling of the cement into a particulate dispersion, following by complexation and infiltration with a resin-based paste or solution, to form a composite-based material. This composite material may be activated with conventional light curing or self-curing chemical initiators in a one or two component material, to form a reinforced calcium-based cement for use as described herein.

A further embodiment of the invention involves the addition of the pre-reacted cement particles, or the pre-cursor unreacted cement powder component, in an acceptable material for endodontic root filling, such as gutta percha. Gutta percha conventionally comes in various forms for a variety of filling techniques using heat to soften and permit the flow of the gutta percha to facilitate the filling and sealing of the root canal space. Alternatively, the pre-reacted cement particles, or the pre-cursor unreacted cement powder component may be added to other conventional thermosensitive or thermo-reversible materials or polymers.

This material complex and its method of application have a wide range of potential indications and clinical uses in dentistry. Potential indications include, but are not limited to: a dental pulp capping agent; a root repair material to correct areas of internal root exposure to the periodontal ligament space; a root canal filling material; an intermediate filling material, liner, or base; a root-end filling material, a bone grafting material; sealing of coronal areas of the root canal filling space to reduce or eliminate coronal leakage; and other restorative, endodontic, or surgical applications in dentistry.

Embodiments of this invention facilitate the creation of an adhesive interface on the surface of calcium-based, biocompatible cements. This unique capability facilitates application and bonding of other adhesive materials and layers, simultaneously or when desired, to the cement and tooth structure. This invention increases the physical unity and stability of the calcium-based, biocompatible, water-based cement to tooth structure and other filling materials.

An additional feature and unique embodiment of this invention is the combination of a self-etching/self-priming adhesive with a water-based cement component. The unique surface infiltration of the bioactive or biocompatible water-based cement with the self-etching adhesive permits a unique clinical utility which permits immediate application of a definitive restorative material over the surface of the hybrid-conjugate material, without delay, while also permitting the continued setting of the water-based cement. In this manner, the application of vital pulp therapy or treatment of endodontic filling procedures can be accomplished in a single, unified procedure. An unanticipated finding of this procedure, when applied in pulp capping, was a high degree of pulpal vitality and pulpal bridge formation when this single step procedure was utilized. The realization of an adhesive, pulp-capping agent was finally achieved.

### Example One

According to the present invention, the use of a calcium-based cement, Mineral Tri-Oxide Aggregate (MTA) available from DENTSPLY International Inc., combined with surface treatment of a self-etching adhesive, Xeno III also from DENTSPLY, provided an effective means to achieve a single-procedure, adhesive pulp capping material and procedure. The procedure was as follows:
1) A cavity preparation is made in a tooth. During the preparation, an exposure of the pulp occurs. Hemostasis (control of bleeding) is accomplished with a cotton pellet soaked in saline. An antimicrobial agent, such as a chlorhexidine preparation (such as Consepsis® from Ultradent Products Inc.), may be also applied to assist in control of bleeding.
2) ProRoot^{™} MTA (DENTSPLY), a calcium containing cement, is then mixed in sterile saline or water and applied carefully to the exposure site.
3) The entire surface of the preparation, including the exposure site, is then treated with a self-etching primer-adhesive, Xeno®. The Xeno® self-etching adhesive is a two-component material, which is mixed prior to application to the cavity preparation. The material is mixed and applied with an appropriate brush or applicator, then lightly air thinned. Other self-etching materials, available commercially, may also be used for this purpose in the procedure.
4) The applied Xeno® self-etching adhesive and the entire preparation are exposed to visible light in the blue spectral range to facilitate curing of the adhesive layer and conjugate Xeno-Adhesive interface.
5) A light-cured, dual-cured, or appropriate self-curing composite restorative (i.e. Spectrum TPH® from DENTSPLY) is applied to fill the cavity preparation and cured with the visible light-curing unit. Appropriate finishing and polishing can then be accomplished to complete the restoration.

When a similar procedure was conducted in an animal experiment, the results indicated an extremely high level of remaining pulpal vitality after the procedure (84.6%), as well as an extremely high level of dentinal bridge formation over the exposed pulp area (84.6%). A zero (0) grade inflammatory reaction was noted for the treated teeth.

### Example Two

An alternative procedure to example one above would be to apply an acetone or ethanol-based, fifth generation, adhesive to the surface of the ProRoot MTA instead of the self-etching adhesive material. In this case, a layer of Prime & Bond NT® (DENTSPLY) would be applied over the surface of the unset ProRoot MTA, lightly air dried, and cured with a visible light curing unit. This procedure would also achieve polymer-cement hybrid interface to stabilize the surface of the MTA or calcium-based cement during setting.

### Example Three

A single component, light-cured material, composed of an appropriate resin matrix material (in this case UDMA), appropriate light-curing initiators, and ground particles of the pre-reacted ProRoot MTA material was prepared for use as a single-component pulp capping agent and dental filling material.

The ProRoot MTA was blended with water, allowed to cure for 48 hours and then ground through an 80 mesh sieve making a premixed product of 83.33% cement with the remainder of water. The premixed and cured cement was combined with UDMA and fumed silica and a photoinitiator as follows.
- Premix Phase 22g
- UDMA 73.2g
- Aerosil 4.6g
- CQ 0.2g
- Total 100.0%

All the ingredients were placed in a vacuum mixer for about 40 minutes. The product was placed into 1.2 cc light impervious syringes.

After achieving hemostasis in an area of pulp exposure, as described above, this single component material described in example 3 was applied to the exposure site with an appropriate applicator and then cured with a visible light-curing unit. The entire cavity preparation was then etched with 34% phosphoric acid, rinsed and damped to be moist without excess water, a layer of Prime & Bond NT was applied to the preparation, lightly air-dried, and cured with a visible light curing unit. The preparation was filled with composite restorative material, Spectrum TPH, and cured with a visible light-curing unit.

When this composition was analyzed histologically, the level of pulpal vitality was very high (91.6%), and the success rate for dentinal bridge formation was also good (75%). The overall inflammatory reaction noted was low - grade 0.5.

The composite pulp-capping material, noted above in this example, may alternatively contain a filler component that is blend of unreacted, ProRoot MTA particles, as well as pre-reacted MTA powder. The two different particle components could be sieved and mixed as a reasonably uniform powder-blend prior to incorporation into the resin matrix material. This powder blend could also be incorporated into other polymer matrix materials, such as gutta percha used in root canal filling procedures.

### Example Four

The pre-reacted cement particles, or the pre-cursor unreacted cement powder component, based on the ProRoot MTA or other calcium-based cement formula, may alternatively be added to an epoxy-based resin root canal sealer composition, AH-Plus® (DENTSPLY). This calcium-based, bioactive powder component material may be added to either or both component pastes prior to or at the site of use of the root canal sealer.

### Example Five

It is known that the initial strength of many calcium-based cements is low and limits their use as temporary or permanent, stress bearing restorative materials. This limitation can be overcome by the present invention, through the application of a polymeric surface layer to the surface of the initially-curing, calcium-based, water-based cement. A wide variety of polymeric coatings and adhesives may be used for this purpose. For example, a temporary filling composed of ProRoot MTA or a calcium aluminate filling material, (just as two examples of calcium-based cements), can be mixed and placed in a cavity preparation. After surface contouring of the cement undergoing initial setting, a polymeric surface material, i.e., Fortify ® or BisCover® (both available from BISCO Dental Products), or any filled or unfilled pit and fissure sealant material, may be brushed or coated over the surface of setting or initially set cement and light cured. This coating stabilizes the surface or the calcium-based, water-based cement and improves its surface hardness and initial strength.

### Example Six

A root canal sealer material or a root canal repair material is made by the combination of a portland or calcium aluminate cement with a solution of 2-10% Polyvinylpyrrolidone. The Polyvinylpyrrolidone may have a molecular weight between 40,000 and 1,300,000.

Other polymers are useful for dental purposes combined with the cement include poly vinyl alcohol, poly vinylacetate, 2-hydroxyethyl methacrylate, sodium n-dodecyl sulfate and/ or phosphorylcholine prepared by mixing equal amount of cationic and anionic polymers.

The combination of these polymer allows the viscosity and setting time to be adjusted as desired for the indication. These polymer enable the cement to set in a matter of minutes as opposed to hours.

Furthermore, the addition of various polymers can increase the compressive strength of the cement from about 50 MPa to over 100 MPa as shown in Table 1.

**Table 1: Compressive strength of PC-gel modified cement**

| Sample # | Compressive strength / MPa |
|---|---|
| 1 | 81 |
| 2 | 102 |
| 3 | 111 |
| 4 | 81 |
| Average | 94 |

Alternatively the cement may contain a radiopacifier such as bismuth oxide, cerium oxide, or a barium or strontium-containing glass. The glass may contain fluoride for fluoride release in vivo.

## Claims

1. A dental composite material comprising a polymer-infiltrated calcium cement, wherein said calcium cement is a mineral trioxide aggregate.

2. A dental composite material as in claim 1, wherein said cement is infiltrated with a polymer selected from the group consisting of water-soluble polymers and hydrophobic resins.

3. A dental composite material as in claim 2, wherein said polymer is selected from the group consisting of UDMA, polyvinylpyrrolidone, polyvinyl alcohol, poly vinylacetate, 2-hydroxyethyl methacrylate, sodium n-dodecyl sulfate and phosphorylcholine prepared by mixing equal amount of cationic and anionic polymers, and mixtures thereof.

4. A dental composite material as in claim 1, further comprising a dental filling material.

5. A dental composite material as in claim 4, wherein said filling material is gutta percha.

6. A dental composite material as in claim 1, having a compressive strength greater than 50 MPa.

7. A dental composite material as in claim 6, having a compressive strength greater than 100 MPa.

8. A dental composite material as in claim 1, wherein said polymer is a dental self- etching adhesive.

9. A dental material as in claim 1, wherein said polymer is a dental self-priming adhesive.

## Patentansprüche

1. Dentalkompositmaterial, umfassend einen mit einem Polymer infiltrierten Kalziumzement, wobei der Kalziumzement ein Mineraltrioxid-Aggregat ist.

2. Dentalkompositmaterial nach Anspruch 1, wobei der Zement mit einem Polymer infiltriert ist, das ausgewählt ist aus der Gruppe, bestehend aus wasserlöslichen Polymeren und hydrophoben Harzen.

3. Dentalkompositmaterial nach Anspruch 2, wobei das Polymer ausgewählt ist aus der Gruppe, bestehend aus UDMA, Polyvinylpyrrolidon, Polyvinylalkohol, Polyvinylacetat, 2-Hydroxyethylmethacrylat, Natrium n-dodecylsulfat und Phosphorylcholin hergestellt durch Vermischen gleicher Mengen von kationischen und anionischen Polymeren, sowie Gemischen davon.

4. Dentalkompositmaterial nach Anspruch 1, das weiterhin einen dentalen Füllstoff enthält.

5. Dentalkompositmaterial nach Anspruch 4, wobei der Füllstoff Guttapercha ist.

6. Dentalkompositmaterial nach Anspruch 1 mit einer Druckfestigkeit von mehr als 50 MPa.

7. Dentalkompositmaterial nach Anspruch 6 mit einer Druckfestigkeit von mehr als 100 MPa.

8. Dentalkompositmaterial nach Anspruch 1, wobei das Polymer ein dentales selbstätzendes Adhäsiv ist.

9. Dentalkompositmaterial nach Anspruch 1, wobei das Polymer ein dentales selbstgrundierendes Adhäsiv ist.

## Revendications

1. Matériau composite dentaire comprenant un ciment au calcium imprégné d'un polymère, dans lequel ledit ciment au calcium est un agrégat de trioxyde minéral.

2. Matériau composite dentaire suivant la revendication 1, dans lequel ledit ciment est imprégné d'un polymère choisi dans le groupe consistant en des polymères hydrosolubles et des résines hydrophobes.

3. Matériau composite dentaire suivant la revendication 2, dans lequel ledit polymère est choisi dans le groupe consistant en UDMA, polyvinylpyrrolidone, alcool polyvinylique, poly(acétate de vinyle), méthacrylate de 2-hydroxyéthyle, n-dodécylsulfate de sodium et phosphorylcholine, préparé en mélangeant des quantités égales de polymères cationique et anionique, et leurs mélanges.

4. Matériau composite dentaire suivant la revendication 1, comprenant en outre une matière d'obturation dentaire.

5. Matériau composite dentaire suivant la revendication 4, dans lequel ladite matière d'obturation est la gutta percha.

6. Matériau composite dentaire suivant la revendication 1, ayant une résistance à la compression supérieure à 50 MPa.

7. Matériau composite dentaire suivant la revendication 6, ayant une résistance à la compression supérieure à 100 MPa.

8. Matériau composite dentaire suivant la revendication 1, dans lequel ledit polymère est un adhésif d'auto-mordançage dentaire.

9. Matériau composite dentaire suivant la revendication 1, dans lequel ledit polymère est un adhésif d'auto-accrochage dentaire.
